Europäisches Patentamt

European Patent Office

Office européen des brevets

(11) Publication number: **0 098 916**
A2

# EUROPEAN PATENT APPLICATION

(21) Application number: **83102393.2**

(22) Date of filing: **16.10.79**

(51) Int. Cl.³: **A 61 L 9/12**
B 65 D 21/02, B 65 D 1/02

(30) Priority: **18.10.78 US 952386**
**13.08.79 US 65848**

(43) Date of publication of application:
**25.01.84 Bulletin 84/4**

(84) Designated Contracting States:
**CH DE FR GB NL SE**

(60) Publication number of the earlier application
in accordance with Art. 76 EPC: **0 019 626**

(71) Applicant: **GEORGIA-PACIFIC CORPORATION**
**133 Peachtree Street, N.E.**
**Atlanta Georgia 30303(US)**

(72) Inventor: **De Luca, Raymond F.**
**15, Whittaker Street**
**Stamford Connecticut 06902(US)**

(74) Representative: **Patentanwälte Wenzel & Kalkoff**
**Ruhrstrasse 26 Postfach 2448**
**D-5810 Witten(DE)**

(54) Dispensing container for an evaporative air freshener.

(57) A dispensing container has a bottom wall, an enclosed side wall, an elongated protusion formed in the side wall, an elongated recess formed in the side wall substantially opposite said protusion, a top wall and an outlet formed in the top wall and in this top wall a sloped portion is integrated which slopes downwardly and outwardly from the outlet. During the use of this container in an inverted position above and close to a reservoir the sloped portion accelerates an air flow which is forced through the gap between the container and the reservoir. This acceleration of the air flow leads to a higher evaporation effect due to the reduced pressure in this area. In this way a liquid material may be used which is less sensitive to temperatures and humidity as to its volatility.

FIG. 1

EP 0 098 916 A2

⌐

Applicant:    Georgia-Pacific Corporation
0900 S.W. Fifth Avenue
Portland, OR 97204
U.S.A.

TITLE MODIFIED
see front page

Title:        A Dispensing Container

The invention relates to a dispensing container comprising a bottom wall, an enclosed  side wall joined to
said bottom wall, an elongated protusion formed in said
side wall, an elogated recess formed in said side wall
substantially opposite said protusion, a top wall joined
to said side wall and an outlet formed in said top wall
and is divided out of the European parent application
79901367.7.

Such dispensing containers are used in air freshener
devices for replenishing the reservoir from which an
evaporating material is dispensed into the ambient.
The container is normally positioned above and in close
proximity to the reservoir. An air stream is forced
for instance by virtue of a motor-driven fan through
the gap between the reservoir and the container to
effect evaporation of the liquid material from the
surface in the reservoir and  directly entrain the
vaporised  material in the air flow.

Even though there is a close contact of the air flow with the liquid surface in the reservoir the rate of evaporation is sometimes insufficient so that a corresponding low release of fragrance is dispensed from the device. A higher volativity of the liquid material is not always recommended to overcome the deficiencies since the evaporation is then too sensitive as to temperature or humidity changes. Accordingly, it is the object of this invention to improve a steady and sufficient evaporation of the liquid material in the reservoir in a dispensing device by altering the air flow over the liquid surface.

The invention is characterized in that the top wall comprises a sloped portion which slopes downwardly and outwardly from said outlet.

The sloped portion is crossed by the forced air flow from the side wall containing the protusion to the outlet in the top wall so that the air flow is forced through a narrowing gap. Because the lower part of this gap consists of the liquid surface or a louvered lid on the reservoir being either horizontal or less inclined than the sloped portion in the top wall of the container. The decreasing gap accelerates the forced air flow and as a result of this acceration there is a pressure drop which enhances the evaporation effect. In this way the volativity of the liquid material need not be too high since        the low pressure area close to the evaporation surface ascertains a sufficient rate of evaporation.

3

The dispensing container according to this invention is a one-way article which is replaced by a fresh container without being refilled. All users of a air refreshener device for which the container according to the invention is adapted to are therefore forced to keep a certain reserve of containers if the air refreshening is expected to be without interruption. In order to save space, preferably the protusion and the recess are substantially parallel. There is no space wasting between the single reserve containers since all side walls are mainly parallel to each other thus allowing a dense packing. These benefits are further enhanced if the protusion and the recess extend substantially along the height of the container. Further increments of space saving are achieved when the protusion and the recess intersect the bottom wall and the top wall of the container.

If space saving is really at a premium the recess may be devi ed larger than the protusion so that a plurality of the containers can be compactly nested for storage with a protusion of the container nesting within the recess of an ädjacent container.

The initially mentioned gap in which the forced air flow is accelerated is rather long and contributes then to a good accelerating effect when the sloped portion is joined to the protusion. Because in this configuration the air flow is accelerated from the protusion up to the outlet approximately in the center of the container. In order to prevent any obstructions against this accelerated air flow an annular collar is formed in the top wall which surrounds the outlet. The necessary support for the inverted container is thus reduced to a minimum which does not compromise the evaporation effect caused by the acceleration of the air flow.

Embodiments of the invention are below described in greater detail by virtue of the drawings in which

Fig. 1    is a perspective view of one embodiment of the dispenser container;

Fig. 2    is a bottom view of the container in Fig. 1;

Fig. 3    shows a plurality of dispenser containers in nested relationship;

Fig. 4 - 6    depict an alternative embodiment of the dispensing container in accordance with the invention.

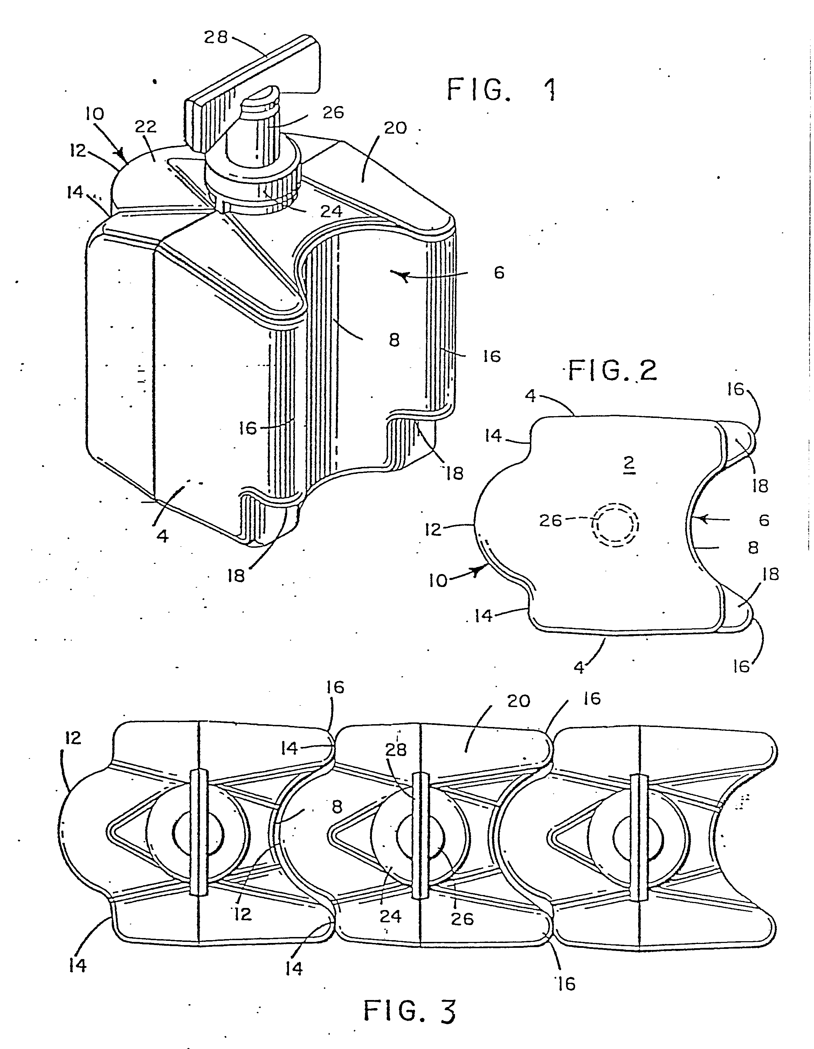

Referring to figures 1 - 3, material supply container is molded of high density plyethylene and has a bottom wall 2 joined to two pairs of opposed side walls. Side walls 4 are substantially flat and parallel while side wall 6 comprises a central recess 8. Side wall 10 on the other hand, comprises a central protusion 12 which is flanked by a pair of shoulders 14. Recess 8 is flanked by a pair of ridges 16 which extend less than the full length of the container to define shoulders 18. Top wall 20 comprises a sloped portion 22 adjacent protusion 12. An annular collar 24 surrounds an outlet tube 26. A break-off closure 28 closes outlet tube 26.

This configuration of opposed recess and protusion enables a plurality of these supply containers to be nested for compact storage as illustrated in figure 3. Collar 25 functions to support the container in an inverted position on a central flange (not shown) with outlet tube 26 extending through this flange into fluid communication with a material in a reservoir below.

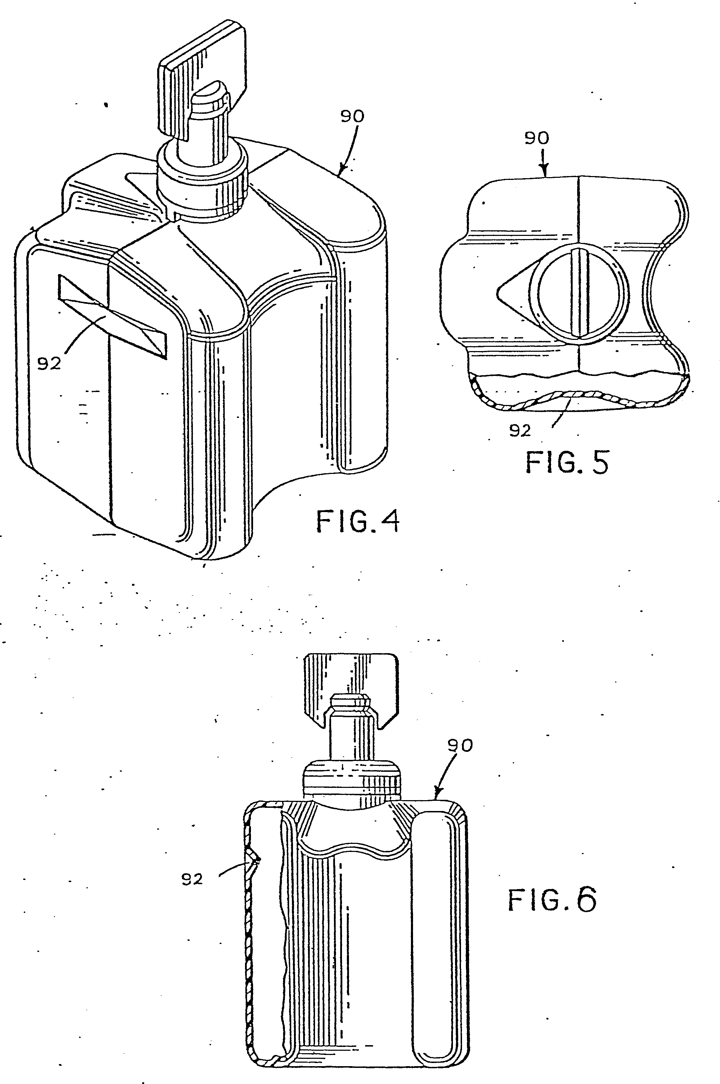

Alternative embodiment of the supply container is illustrated in figures 4, 5 and 6. Container 90 is generally similar in capacity , size and shape to the afore mentioned container and is also nestable with this of the same type.

However, container 90 is devoid of shoulders 18, because an alternative container engaging means in the form of two opposed dimples 92 are formed in the container wall. Dimples 92 are engaged  by snap tabs (not shown) which firmly retain container 90 in position.

In each embodiment the slopedportion 22 is noticeable reaching from protusion 12 to the center outlet tube 26 . This slopedportion 22 causes an air flow to accelerate when the container is positioned on top of a reservoir in an inverted position, i. e. with the outlet tube 26 facing downwards. Then the slopedportion 22 forms a decreasing gap for an air flow which is directed from the side of the container to its center over the slopedportion 22.

Patent Claims

1. A dispenser container comprising a bottom wall (2), an enclosed side wall (4, 6,10) joined to said bottom wall (2), an elongated protusion (12) formed in said side wall (10),an elongated reset (8) formed in said side wall (6) substantially opposite said protusion (12), a top-wall (20) joined to said side wall (4,6,10), and an outlet (26) formed in said top-wall (20) c h a r a c t e r i z e d in that said top-wall (20) comprises a sloped portion (22) which slopes downwardly and outwardly from said outlet (26).

2. A container according to claim 1 w h e r e i n said protusion (12) and said recess (8) are substantially parallel.

3. A container according to claim 2 w h e r e i n said protusion (12) and said recess (8) extend substantially along the hight of the container.

4. A container according to calim 3, w h e r e i n said protusion (12) and said recess (8) intersect said bottom wall (2) and said top-wall (20).

5. A container according to claim 4, w h e r e i n said recess (8) is larger than said protusion (12) so that a plurality of the containers can be compactly nested for storage with the protusion (12) of one container nesting within the recess (8) of an adjacent container.

6. A container according to claim 5 w h e r e i n said sloped portion (22) is joined to said protusion (12).

7. A container according to claim 6 w h e r e i n a protuding annular collar (24) is formed in said top wall (20) which surrounds said outlet (26).

FIG. 1

FIG. 2

FIG. 3

FIG.4

FIG.5

FIG.6